# EUROPEAN PATENT SPECIFICATION

(11) **EP 3 251 701 B1**
(45) Date of publication and mention of the grant of the patent: **27.11.2019**
(21) Application number: 16842208.7
(22) Date of filing: 26.08.2016
(51) Int. Cl.: A61K 49/04, A61K 9/48, A61K 47/30, A61B 5/00

(54) **MEDICAL AID FOR INTESTINE EXAMINATIONS**
MEDIZINISCHE HILFE FÜR DARMUNTERSUCHUNGEN
ACCESSOIRE MÉDICAL POUR EXAMENS DE L'INTESTIN

(30) Priority: 01.09.2015 KR 20150123540
(43) Date of publication of application: 06.12.2017
(73) Proprietor: Kim, Chang Bo, Guri-si, Gyeonggi-do 11917 (KR)
(72) Inventor: Kim, Chang Bo, Guri-si, Gyeonggi-do 11917 (KR)
(74) Representative: Müller-Boré & Partner Patentanwälte PartG mbB
(86) International application number: PCT/KR2016/009533
(87) International publication number: WO 2017/039242

(56) References cited:
- JP-A- 2002 071 669
- KR-A- 20010 045 671
- KR-A- 20080 018 185
- KR-A- 20080 079 421
- KR-A- 20100 071 258
- KR-B1- 101 061 224
- US-A1- 2009 118 579

## Description

### [TECHNICAL FIELD]

The present invention relates to a medical aid for intestine examinations that may improve the reliability of an examination by accurately and easily identifying a pass time of an intestine according to lapse of time when the motility of the intestine is examined, thereby achieving an accurate diagnosis and an accurate prescription.

### [BACKGROUND ART]

A large intestine is a tubular organ that corresponds to a final part of a gastrointestinal tract starting from the appendix and ending at the anal passage, and is a part in which diseases such as colitis ulcerativa, local colitis, and polypus are caused.

Generally, in order to accurately examine the state of a large intestine by using X-rays, a contrast medium that may form a proper contrast against surrounding body tissues are injected for X-ray photographing.

Meanwhile, medical aids for photographing and measuring the motility of an intestine of the constipation patients or the patients whose intestine motilities deteriorate with radioactive rays have been suggested, and the medical aids are materials that are not biodegraded in the human body, are not harmful to the human body, maintain the properties and forms thereof from the initial state after being taken, and do not change even in the case of defecation after a long-time stay in the large intestine, and are taken for three days at an interval of 24 hours after they are taken at the first time and the medical aids left after radioactive photographing measured after four to seven days.

However, the medical aids according to the related art are simple and uniform so that the shapes for dates when the medical aids are taken cannot be discriminated. That is, because the conventional medical aids are concealed according to the shapes and sizes thereof when they are photographed and are photographed while the shapes thereof are the same, they cannot be individually identified so that an accurate measurement according to the intestine pass time is difficult.

### [Prior technical documents]

(Patent document 1) (Document 1) Korean Patent Application Publication No. 2001-0045671 (entitled "Recording Material for Radioactive Examinations" and published on June 5, 2001)
(Patent document 2) (Document 2) Korean Patent No. 10-1061224 (entitled "Capsule for Measuring Flow Information using X-rays" and published on August 25, 2011). Document JP2002071669 discloses biodegradable capsules filled with ring shaped markers made of barium sulfate.

### [DETAILED DESCRIPTION OF THE INVENTION]

### [TECHINICAL PROBLEM]

Accordingly, the present invention provides a medical aid for intestine that may improve the reliability of an examination by allowing the shapes taken for dates for three days to be identified and allowing the shapes to be identified even though they are overlaid with each other to allow the pass time of an intestine according to lapse of time to be accurately and easily identified when the motility of the intestine is measured and examined, thereby achieving an accurate diagnosis and an accurate prescription.

The objectives of the present invention are not limited to the above-mentioned one, and the unmentioned objectives of the present invention will be clearly understood by an ordinary person skilled in the art from the following description.

### [TECHNICAL SOLUTION]

In accordance with an aspect of the present invention, there is provided a medical aid for intestine examination including a plurality of recording/measuring members formed of a polymeric composite containing a radioactive opaque material and having different forms, and a capsule type biodegradable accommodation body accommodating the recording/measuring members and formed of a material that is biodegraded after being taken.

According to the present invention, the recording/measuring members may be formed by mixing and fusing powder of a polymeric composite of polyethylene and powder that is not harmful to a human body, and performing at least one of a method of injection-molding and a method of extrusion and cutting,

According to the present invention, the powder of the polymeric composite of polyethylene may include low-density polyethylene of polyethylene (C₂H₄(C₂H₄)ₙ) and the power that is not harmful to the human body may include barium sulfate (BaSO₄), and the recording/measuring members may be formed by mixing powder of polyethylene (C₂H₄(C₂H₄)ₙ) and powder of barium sulfate (BaSO₄ at a weight ratio of 4:6 to 6:4, and performing at least one of a method of heating at 130 to 135°C and injection-molding and a method of extrusion and cutting.

According to the present invention, the recording/measuring members may be formed by mixing powder of polyethylene (C₂H₄(C₂H₄)ₙ) and powder of barium sulfate at a weight ratio of 5.5:4.5.

According to the present invention, each of the recording/measuring members may include an annular recording body formed through injection-molding or extrusion and cutting, a ball-shaped recording body formed through injection-molding, and a polyhedral recording body formed through injection-molding or extrusion.

According to the present invention, the sizes of the annular recording body, the polyhedral recording body, and the ball-shaped recording body becomes smaller in their sequence in the recording/measuring member.

According to the present invention, when the ball-shaped recording body and the polyhedral recording body are viewed while being overlaid with each other on a plane with reference to the annular recording body, the distance between the concentric circle to the outermost surface of the annular recording body may be the largest, the distance between the concentric circle to the outermost surface of the polygonal recording body may be the second largest, and the distance between the concentric circle to the outermost surface of the ball recording body may be the smallest.

### [ADVANTAGEOUS EFFECTS OF THE INVENTION]

The medical aid for intestine examinations according to the present invention may improve the reliability of an examination by accurately and easily identifying a pass time of an intestine according to lapse of time when the motility of the intestine is examined, and accordingly, an accurate diagnosis and an accurate prescription may be achieved.

The effects of the present invention are not limited to the above-mentioned one, and the unmentioned effects of the present invention will be clearly understood by an ordinary person skilled in the art from the following description.

### [DESCRIPTION OF THE DRAWINGS]

Fig. 1 is a diagram illustrating a medical aid for intestine examinations according to the present invention;
Fig. 2 is a plan view illustrating a recording/measuring member constituting the medical aid for intestine examinations according to the present invention; and
Fig. 3 is an X-ray picture according to lapse of time after the medical aid for intestine examinations is taken according to the present invention.

### [BEST MODE]

The additional objectives, features, and advantages of the present invention will be understood more clearly from the following description and the accompanying drawings.

When it is mentioned that one element is "connected to" or "electrically connected to" another element, it should be understood that the first element may be directly connected or electrically connected to the second element but a third element may be provided therebetweeen. On the other hand, when it is mentioned that an element is "directly connected to" or "directly electrically connected to" another element, it should be understood that a third element is not present between them.

The terminologies used herein are provided only to describe specific embodiments, and are not intended to limit the present invention. The terms of a singular form may include plural forms unless otherwise specified. The terms "including" and "having" are used to designate that the features, the numbers, the steps, the operations, the elements, the parts, or combination thereof described in the specification are present, and may be understood that one or more other features, numbers, step, operations, elements, parts, or combinations thereof may be added.

Further, the terms, such as "unit", "- er, - or", and "module" described in the specification mean a unit for processing at least one function or operation, and may be implemented by hardware, software, or a combination of hardware and software.

Further, in the description of the present invention with reference to the accompanying drawings, the same reference numerals are given to the same element, and a repeated description thereof will be omitted. A detailed description of known technologies related to the present invention will be omitted to avoid making the technical essence of the present invention rather unclear.

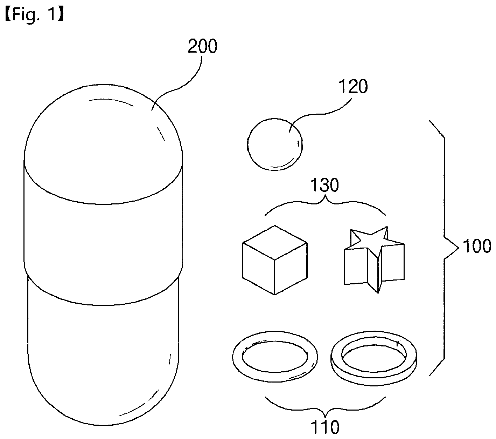

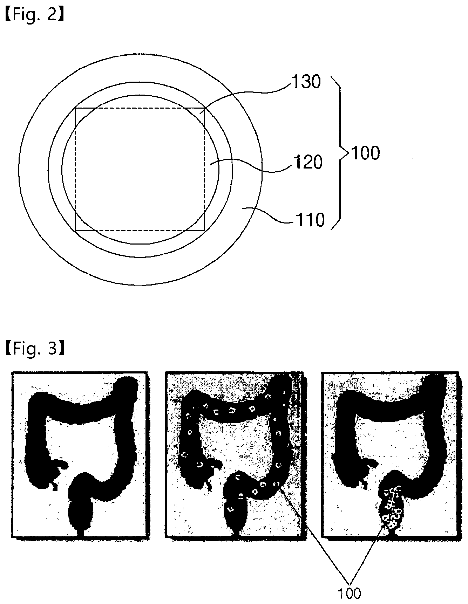

Hereinafter, a medical aid for intestine examinations according to a preferred embodiment of the present invention will be described with reference to Fig. 1. Fig. 1 is a diagram illustrating a medical aid for intestine examinations according to the present invention. Fig. 2 is a plan view illustrating a recording/measuring member constituting the medical aid for intestine examinations according to the present invention.

As illustrated in Fig. 1, the medical aid for intestine examinations according to the present invention if formed of a polymeric composite containing a radioactive opaque material, and includes a plurality of recording/measuring member 100 having different forms; and a biodegradable accommodation body 200 accommodating the recording/measuring member 100 and formed of a degradable material.

The recording/measuring members 100 are formed by mixing powder of a polymeric composite, such as polyethylene, and powder of barium sulfate (BaSO₄), which is a radioactive opaque material that is not harmful to the human body, at a weight ratio of 4:6 to 6:4, preferably, at a weight ratio of 5:4.5 and forming the recording/measuring members 100 into a specific form through heating, injection-molding and/or extrusion.

Here, when the ratio of the polymeric composite powder is less than 4 or the ratio of the barium sulfate powder exceeds 6, the formation performance of the recording/measuring members 100 is not good, and when the ratio of the polymeric composite powder exceeds 6 or the ratio of the barium sulfate powder is less than 4, the readability (discrimination) deteriorates.

According to the present invention, it is preferable that the ratio of the powder of a polymeric composite of polyethylene and the powder of barium sulfate (BaSO₄), which is a radioactive opaque material that is not harmful to the human body, be 5.5:4.5 in consideration of the formation performance and readability (discrimination).

Each of the recording/measuring members 100 includes an annular recording body 110 formed through injection-molding or extrusion and cutting, a ball-shaped recording body 120 formed through extrusion, and a polyhedral recording body 130 formed through injection-molding or extrusion and cutting. Here, the injection-molding is achieved by manufacturing the recording/measuring members 100 while maintaining the external shapes thereof such that the sizes and shapes of the recording/measuring members 100 correspond to the original sizes and shapes thereof, and the recording/measuring members 100 are manufactured into the original sizes thereof by extruding the material into the shapes of a rod and cutting the rod by a specific length.

Although Fig. 1 illustrates that the annular recording body 110 is a circular ring, the present invention is not limited thereto but any annular shape is possible. That is, any annular shape having a circular, tetragonal, or polygonal section and having a circular space in the interior of the annular shape is possible.

Further, although the ball-shaped recording body 120 is a sphere, any circular sectional shape including an elliptical shape and accommodated in the space in the circular ring of the annular recording body 110 is possible.

Next, although it is most preferable that the polygonal recording body 130 is a hexahedron, any polyhedral shape, such as an octahedron, a dodecahedron, or a polyhedral shape having a section of star shape, having corner angles on the outside thereof is possible. Here, it is preferable that the contour of the outer line of the polyhedral recording body 130 be viewed clearly when the polyhedral recording body 130 is overlaid with the ball-shaped recording body, by making the size of the polyhedral recording body 130 large enough to be accommodated in the space in the circular ring.

In a description of the sizes of the annular recording body 110, the ball-shaped recording body 120, and the polyhedral recording body 130 constituting the recording/measuring member 100, as illustrated in Fig. 2, the sizes of the annular recording body 110, the polyhedral recording body 130, and the ball-shaped recording body 120 become smaller in their sequence. In other words, when the ball-shaped recording body 120, and the polyhedral recording body 130 are viewed while being overlaid with each other on a plane with reference to the annular recording body 110, the distance between the concentric circle to the outermost surface of the annular recording body 110 is the largest, the distance between the concentric circle to the outermost surface of the polygonal recording body 130 (the apex thereof may be viewed in Fig. 2) is the second largest, and the distance between the concentric circle to the outermost surface of the ball recording body 120 is the smallest. Then, it is preferable that the sizes of the outer sides of the polyhedral recording body 130 and the ball-shaped recording body 120 be smaller than the diameter of the ring of the annular recording body 110 such that the polyhedral recording body 130 and the ball-shaped recording body 120 may be accommodated in the annular recording body 110 while having a slight gap therebeween and the apexes of the outer corners of the polyhedral recording body 130 be exposed to the outside of the ball-shaped recording body 120 to be viewed through the gap when the polyhedral recording body 130 and the ball-shaped recording body 120 are overlaid with each other.

That is, the recording/measuring member 100 has the size differences, and thus even though the recording/measuring member 100 is overlaid in any direction, in particular, even though the recording/measuring member 100 is overlaid on a plane as illustrated in Fig. 2, the contour of the external appearance of the recording/measuring member 100 may be easily discriminated with an X-ray picture.

Next, the polymeric composite of polyethylene is polyethylene (C₂H₄(C₂H₄)ₙ), and polyethylene (C₂H₄(C₂H₄)ₙ) has no impurities and has no harmful property, is strong to the acidity of digestive juice, and has a melting point of 132 to 135°C. Then, it is preferable that the polymeric composite of polyethylene be LDPE.

The biodegradable accommodation body 200 is a material that is not harmful to the human body and may be biodegraded after being taken. In other words, the biodegradable accommodation body 200 refers to a capsule that is well known in the medical field and may be sufficiently understood by an ordinary person skilled in the art, and thus a detailed description thereof will be omitted. Because 20 to 24 pills of the recording/measuring members 200 are generally taken by the patient, the patient may easily take the recording/measuring members 200 through the biodegradable accommodation body 200 without feeling foreign substances.

The biodegradable accommodation body 200 is biodegraded if reaching the intestine through the esophagus, and the recording/measuring members 100 accommodated therein are introduced into the small intestine and the large intestine while the forms thereof is maintained.

Fig. 3 is an X-ray picture according to lapse of time after the medical aid for intestine examinations is taken according to the present invention.

If about twenty to twenty four recording/measuring members 100 are taken at an interval of twenty four hours for three days, for example, in the sequence of the annular recording body 110, the ball-shaped recording body 120, and the polyhedral recording body 130 in order to examine the motility of an intestine by using the medical aid for intestine examination according to the present invention, the examination of the intestine, for example, the motility of the intestine or constipation may be recognized by identifying at which location of the intestine which recording/measuring member is located as illustrated in FIG. 2.

The medical aid for intestine examinations according to the present invention may improve the reliability of an examination by accurately and easily identifying a pass time of an intestine according to lapse of time when the motility of the intestine is examined, and accordingly, an accurate diagnosis and an accurate prescription may be achieved.

### [Description of Reference Numerals]

100: recording/measuring member
110: annular recording body
120: ball-shaped recording body
130: polyhedral recording body
200: biodegradable accommodation body

[INDUSTRIAL APPLICABILITY]

## Claims

1. A medical aid for intestine examination comprising:
a plurality of recording/measuring members (100) formed of a polymeric composite containing a radioactive opaque material and having different forms; and
a capsule type biodegradable accommodation body (200) accommodating the recording/measuring members (100) and formed of a material that is biodegraded after being taken,
wherein the recording/measuring members are formed by mixing powder of polyethylene (C₂H₄(C₂H₄)ₙ) and powder of barium sulfate (BaSO₄) at a weight ratio of 4:6 to 6:4, and performing at least one of a method of heating at 130 to 135°C and injection-molding and a method of extrusion and cutting,
wherein each of the recording/measuring members (100) includes an annular recording body (110), a ball-shaped recording body (120), and a polyhedral recording body (130), and when the ball-shaped recording body (120) and the polyhedral recording body (130) are viewed while being overlaid with each other on a plane with reference to the annular recording body (110), the distance between the concentric circle to the outermost surface of the annular recording body (110) is the largest, the distance between the concentric circle to the outermost surface of the polygonal recording body (130) is the second largest, and the distance between the concentric circle to the outermost surface of the ball recording body (120) is the smallest, and
wherein a distance from the ball-shaped recording body (120) to an outermost surface of the polygonal recording body (130) is smaller than a diameter of a circular space in a ring of the annular recording body (110) such that a gap for accommodation is formed.

## Patentansprüche

1. Medizinische Hilfe zur Darmuntersuchung, umfassend:
eine Mehrzahl von Aufzeichnungs-/Messelementen (100), die aus einem Polymerverbundstoff gebildet sind, der ein radioaktives opakes Material enthält und unterschiedliche Formen aufweist; und
einen biologisch abbaubaren Aufnahmekörper (200) vom Kapseltyp, der die Aufzeichnungs-/Messelemente (100) aufnimmt und aus einem Material besteht, das nach der Entnahme biologisch abgebaut wird,
wobei die Aufzeichnungs-/Messelemente durch Mischen von Polyethylenpulver (C₂H₄(C₂H₄)ₙ) und Bariumsulfatpulver (BaSO₄) in einem Gewichtsverhältnis von 4:6 bis 6:4 und Durchführen von zumindest einem eines Erwärmungsverfahrens bei bzw. auf 130 bis 135°C und Spritzgießen sowie Extrusions- und Schneideverfahren gebildet werden,
wobei jedes der Aufzeichnungs-/Messelemente (100) einen ringförmigen Aufzeichnungskörper (110), einen kugelförmigen Aufzeichnungskörper (120) und einen polyedrischen Aufzeichnungskörper (130) enthält, und wenn der kugelförmige Aufzeichnungskörper (120) und der polyedrische Aufzeichnungskörper (130) betrachtet werden, während sie in einer Ebene in Bezug auf den ringförmigen Aufzeichnungskörper (110) einander überlagert sind, der Abstand zwischen dem konzentrischen Kreis zu der äußersten Oberfläche des ringförmigen Aufzeichnungskörpers (110) am größten ist, der Abstand zwischen dem konzentrischen Kreis zu der äußersten Oberfläche des polygonalen Aufzeichnungskörpers (130) am zweitgrößten ist, und der Abstand zwischen dem konzentrischen Kreis zu der äußersten Oberfläche des Kugelaufzeichnungskörpers (120) am kleinsten ist, und
wobei ein Abstand von dem kugelförmigen Aufzeichnungskörper (120) zu einer äußersten Oberfläche des polygonalen Aufzeichnungskörpers (130) kleiner ist als ein Durchmesser eines kreisförmigen Raumes in einem Ring des ringförmigen Aufzeichnungskörpers (110), so dass ein Spalt zur Aufnahme gebildet wird.

## Revendications

1. Accessoire médical pour examen intestinal comprenant :
une pluralité d'éléments d'enregistrement/mesure (100) formés en un composite polymère contenant un matériau opaque radioactif et ayant différentes formes ; et
un corps d'hébergement biodégradable de type capsule (200) hébergeant les éléments d'enregistrement/mesure (100) et formé en un matériau qui est biodégradé après avoir été capturé,
dans lequel les éléments d'enregistrement/mesure sont formés en mélangeant de la poudre de polyéthylène (C₂H₄(C₂H₄)ₙ) et de la poudre de sulfate de baryum (BaSO₄) à un rapport pondéral de 4/6 à 6/4, et réalisant au moins un procédé de chauffage à 130 à 135°C et de moulage par injection et un procédé d'extrusion et de découpe,
dans lequel chacun des éléments d'enregistrement/mesure (100) inclut un corps d'enregistrement annulaire (110), un corps d'enregistrement en forme de balle (120) et un corps d'enregistrement polyédrique (130), et lorsque le corps d'enregistrement en forme de balle (120) et le corps d'enregistrement polyédrique (130) sont visualisés tout en étant superposés l'un avec l'autre sur un plan en référence au corps d'enregistrement annulaire (110), la distance entre le cercle concentrique et la surface externe du corps d'enregistrement annulaire (110) est la plus grande, la distance entre le cercle concentrique et la surface externe du corps d'enregistrement polygonal (130) est la seconde plus grande, et la distance entre le cercle concentrique et la surface externe du corps d'enregistrement en forme de balle (120) est la plus petite, et
dans lequel une distance du corps d'enregistrement en forme de balle (120) à une surface externe du corps d'enregistrement polygonal (130) est inférieure à une distance d'un espace circulaire dans un anneau du corps d'enregistrement annulaire (110) de sorte qu'un espace pour hébergement est formé.
